# EUROPEAN PATENT APPLICATION

(11) **EP 4 018 927 A1**
(43) Date of publication of application: **29.06.2022**
(21) Application number: 21217630.9
(22) Date of filing: 23.12.2021
(51) Int. Cl.: A61B 5/08, A61B 5/00, G01N 33/497

(54) **APPARATUS FOR IDENTIFYING PATHOLOGICAL STATES AND CORRESPONDING METHOD.**

(30) Priority: 23.12.2020 IT 202000032249
(71) Applicant: Eurotech S.P.A., 33020 Amaro (UD) (IT)
(72) Inventor: Padulosi, Ugo, 33020 Amaro (UD) (IT); Dal Ben, Mattia, 33020 Amaro (UD) (IT); Tamigi, Matteo, 33020 Amaro (UD) (IT); Querin, Sandra, 33020 Amaro (UD) (IT); Adami, Stefano, 33020 Amaro (UD) (IT)
(74) Representative: Petraz, Gilberto Luigi

(57) **Abstract**

The present invention concerns an apparatus for identifying pathological states of a subject comprising at least a set of detection devices, a data processing module and a data communication module and/or a data saving module. The present invention also concerns a platform for identifying pathological states, a method for training an identification apparatus and a method for identifying pathological states.

## Description

### FIELD OF THE INVENTION

The present invention concerns an apparatus and a method for identifying pathological states by analyzing the breath of a patient.

The invention is particularly suitable for identifying respiratory pathologies, although it is not limited to these.

The invention also concerns a platform for identifying pathological states comprising a training system for an identification apparatus.

### BACKGROUND OF THE INVENTION

It is known that many pathological states lead to the presence, in the air exhaled by a subject, of volatile substances, in particular volatile organic compounds (VOCs), in concentrations higher than those that correspond to a physiological level. In these cases, it is possible to diagnose intolerances, respiratory type pathologies, diabetes, some tumors and other types of pathology.

The exhaled air can be collected and analyzed using laboratory methods, such as gas chromatography with mass spectrometry, ion mobility spectrometry or suchlike. These methods of analysis usually have long waiting times and high costs. Furthermore, the measuring instrumentation is usually a laboratory instrumentation, of not negligible size and not transportable.

Some research and state-of-the-art solutions report the application of sets, or arrays, of sensors, for detecting volatile compounds. In this way, it is possible to create instruments transportable and cheaper than laboratory instruments, but they have the disadvantage of low repeatability and accuracy of the measurements.

US 2020/029858 A1 describes a system, a method and a device for detecting, identifying and measuring the concentration of particular substances, such as volatile organic compounds (VOCs), volatile gases and ketones, in a subject's breath. It also provides to use the concentration values of the substances as above to identify a pathological condition. The system can comprise a sensor or several sensors for measuring said concentrations.

Document US 2010/137733 A1 describes a method for diagnosing asthma, tuberculosis and lung cancer, in particular by detecting and measuring the concentration of volatile organic compounds (VOCs) and possibly nitric oxide (NO), in the breath. The method provides to use a system for measuring the substances as above comprising a gas chromatograph and a detection array. The system can also comprise a spirometer, for measuring the volume of exhaled air.

Document US 2019/125211 A1 describes a portable device and a method for detecting and identifying one or more compounds in a subject's breath, such as drugs or compounds produced by drug degradation.

There is therefore a need to perfect an apparatus for identifying pathological states starting from the breath of a subject that can overcome at least one of the disadvantages of the state of the art.

In particular, one purpose of the present invention is to make available an apparatus for identifying pathological states that allows to increase the reliability of the diagnosis obtained, understood as the percentage of false positives and/or false negatives on the total number of diagnoses.

It is also a purpose of the invention to allow continuous updating of the identification apparatus as new data and information relating to the pathological states become available.

Another purpose of the present invention is to provide an apparatus for identifying pathological states which requires reduced development and updating times compared with current solutions.

Yet another purpose is to require, for processing and analyzing the data, limited computing resources from the point of view of computational capacity, the associated economic aspect and further aspects such as compactness, portability and consumption of the calculation devices associated with the apparatus.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, some embodiments described here concern an apparatus for identifying pathological states and a corresponding method, which overcome the limits of the state of the art and eliminate the defects present therein.

The present invention can be applied to identify pathological states by analyzing the breathing of a subject, in particular but not exclusively for respiratory diseases, for example those resulting from COVID19 and Tuberculosis.

Some embodiments described here also concern a platform for identifying pathological states comprising the identification apparatus as above.

Some embodiments described here also concern a method for training an apparatus for identifying pathological states.

In accordance with some embodiments, the apparatus for identifying pathological states comprises, as main components, at least a set of detection devices and a data processing module which can be trained by means of artificial intelligence techniques.

The set of detection devices as above comprises devices chosen from broadband gas devices configured to analyze the air exhaled by the subject and provide signals associated with the concentration of volatile substances present in the exhaled air and/or detection devices for monitoring breathing.

In this description, a broadband sensor means a non-selective sensor, which therefore does not perform a chemical speciation of the analyzed odor and does not necessarily identify the individual molecules that compose it. In particular, a broadband sensor is a sensor that can have a higher sensitivity for one molecule or compound and a lower sensitivity for other molecules or compounds, for example ones that are similar or of the same type.

The set of detection devices as above comprises at least two of either detection devices of a different type and/or detection devices configured to function in different operating conditions.

The detection devices of a different type can be at least two broadband gas devices sensitive to different types of gas, at least two detection devices for monitoring breathing sensitive to different parameters, or at least one broadband gas device and at least one detection device for monitoring breathing or similar cases. The different operating conditions as above concern detection devices that are characterized differently or that work under different conditions of temperature, pressure, breath flow, time and/or suchlike.

The identification apparatus also comprises a data communication module and/or a data saving module.

The identification apparatus also comprises a chamber for containing the air exhaled by the subject, in which at least the set of gas sensors is positioned, the containing chamber comprising an inlet and an outlet for the air.

The broadband gas sensors are configured to analyze a flow of air exhaled by the subject and provide a signal associated with the concentration of one or more volatile inorganic and organic compounds present in the exhaled air.

The processing module can be trained to process at least the signals as above and to synthesize an indicator of the classification of the subject in a class, among a set of classes to which the pathological state belongs. The classes of belonging identify levels of severity relating to the pathological state with respect to which a diagnosis for the subject is to be made.

The data communication module is configured to send at least the signals, and possibly the classification indicator, to a distributed electronic architecture outside the identification apparatus, and to update the processing module.

In this way, it is possible to make the identification apparatus communicate with the outside, even in real time, for example to send to the processing module algorithms that identify pathological states that are constantly updated with the information and/or signals and/or indicators gradually processed. The identification apparatus can therefore be updated continuously, possibly even during the same step of identifying the pathological state, with re-trained and therefore updated identification algorithms.

Advantageously, the use of broadband gas detection devices, not specific for each molecule and which do not require accurate calibrations, allows to achieve reduced purchase and maintenance costs.

In particular, the signal of the sensors as above is not used to measure the concentration of particular substances.

Advantageously, the apparatus according to the invention is also suitable for use with pathologies for which the correlation with the presence of certain substances, and in particular at certain concentrations, in the breath is not known. In this way, it can also be used for diseases for which there are no clinical studies or statistical or epidemiological data, for example because they are new.

The data processing module, which can be trained by means of artificial intelligence techniques, also allows to use all the information associated with the signals provided by the detection devices, allowing to overcome the low specificity of the detection devices through the redundancy of the signals acquired and/or the cross-correlation between the quantity of different volatile substances in the air.

Furthermore, the analysis performed by means of the data processing module, which can be trained by means of artificial intelligence techniques and can also be updated continuously, allows to adapt the processing of the signals to the deviations over time of the detection devices, without the need for continuous or frequent maintenance, for example for calibration operations.

The use of artificial intelligence techniques can also allow to prevent the need to normalize how the air is exhaled by the different subjects, both in the method for training the identification apparatus, and also in the identification method, where the exhaled air is analyzed by the trained identification apparatus.

In particular, the processing module as above can be trained to recognize the fingerprint (pattern recognition) of the pathological state, understood as an identifying and recognizable sign of the pathological state, by means of a "breath fingerprint".

With breath fingerprint we mean an identifying and recognizable sign of the exhaled air associated with the type and characteristics of the substances exhaled (not known a priori) and/or the modalities of the exhalation (fast, slow, difficult and suchlike) and/or the physical characteristics of the exhaled air (temperature, humidity or suchlike).

Advantageously, the same identification apparatus can be used both to train the data processing module and also to identify pathological states.

This allows to reduce the development and initial validation times before the definition and mass production of an identification apparatus that is specialized and optimized for the sole identification of interest. Advantageously, it is therefore possible to reduce update times and increase reliability, since no further sources of uncertainty are introduced, caused by the use of different instruments and methods for acquiring signals and for processing them in the two different steps.

According to one embodiment, the identification apparatus further comprises detection devices for monitoring breathing selected from a group comprising acoustic sensors, flow and/or flow rate sensors, sensors for detecting the motion of the rib cage of the subject, temperature sensors and/or humidity sensors, fine particulate sensors.

In this way, it is also possible to analyze physical characteristics of the exhaled air, selected from a group comprising a duration, an exhalation flow or exhalation profile of the patient. Analyzing the physical characteristics of the exhaled air of the patient can integrate the information content of the exhaled air: the dynamics of the subject's exhalation can be indicative of particular pathologies, in particular pathologies of the respiratory type.

The identification apparatus can comprise a local interface to instruct the subject on how to exhale the air, during one or more exhalation steps. In this way, it is possible to detect volatile compounds that are released only at determinate moments of the exhalation, such as for example during the part of maximum emptying of the lung volume.

Exhaled air acquisition techniques, for example in spirometry, adopt exhalation maneuvers that involve zones of the lungs, such as the alveoli, which due to the pathological state being analyzed can carry significant volatile substances in the exhaled air. One example is the content of substances in the exhaled air during the "end-tidal", that is, the air at the end of the exhalation.

The optimal profile of the exhaled air to be adopted can vary from one pathological state to another. For example, in some pathologies, such as in symptomatic cases of COVID 19, it could be difficult to follow the instructions of exhaling "for a long time".

The identification apparatus according to the invention can instead lead to a more significant exhalation, for example by signaling to the patient, based on the measurements made, to exhale more or less intensely.

The identification apparatus according to the invention can comprise at least one device for pre-processing the indicators provided by the detection devices, configured to pre-process the signals of the detection devices to be represented, as images, in two dimensions. In this case, the data processing module can be able to be trained to recognize images of objects. Advantageously, the image training of the processing module can require, for data processing and analysis, more limited computing resources than the processing of the raw signals provided by the detection devices, from the point of view of both computing capacity and also the associated economic aspect.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic representation of an apparatus for identifying pathological states according to some embodiments;
- fig. 2 is a schematic representation of a platform for identifying pathological states comprising the apparatus for identifying pathological states of fig. 1, according to some embodiments;
- fig. 3 is a schematic representation of a method for training the apparatus for identifying pathological states of fig. 1, according to some embodiments;
- fig. 4 is a schematic representation of a method for identifying pathological states by means of the apparatus for identifying pathological states of fig. 1.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the possible embodiments of the invention, of which one or more non-limiting examples are shown in the attached drawings. The phraseology and terminology used here is also for the purposes of providing non-limiting examples.

Some embodiments described here concern an apparatus 10 for identifying pathological states, and a corresponding method 200.

The invention can be applied to identify pathological states by analyzing the breathing of a subject 300, in particular but not exclusively, for respiratory diseases, for example resulting from COVID19 and Tuberculosis.

Some embodiments described here also concern a platform 50 for identifying pathological states comprising the identification apparatus 10.

The invention also concerns a method 100 for training an apparatus 10 for identifying pathological states.

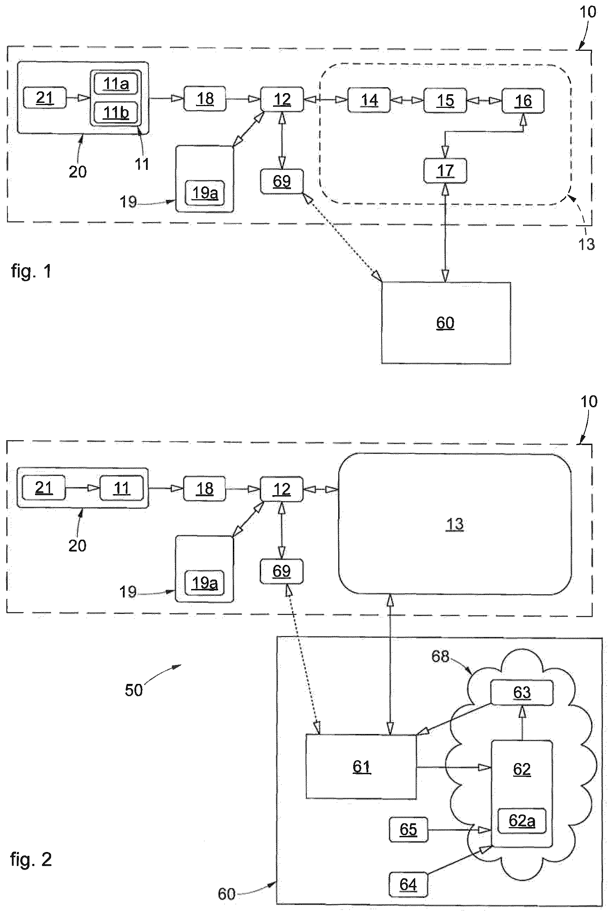

As shown in fig. 1, an apparatus 10 for identifying pathological states comprises at least a set of detection devices 11, a data processing module 12 which can be trained by means of artificial intelligence techniques, a data communication module 13 and a data saving module 69.

According to some embodiments, the same identification apparatus 10 can be used both to train the data processing module 12 and also only to identify and diagnose pathological states.

According to some variants, the identification apparatus 10 for identification and diagnosis only is constructively and/or functionally different from the identification apparatus 10 for training and collection of data from breathing.

In the identification apparatus 10 for identification and diagnosis only, the acquisition and communication part can be simplified, since it is used only as a diagnostic tool, by pre-loading the identification algorithms, for example a trained neural network, at the time of production, and not be able to be updated again in real time. In this case, the identification apparatus 10 for identification and diagnosis only can contain a simplified data processing module 12 and/or not contain a data communication module 13, or contain a simplified data communication module 13, for example without a database, visualization and communication software.

In addition, or alternatively, the identification apparatus 10 for identification and diagnosis only can have a simplified contagion protection part, which can be used as a personal device for use similar to a mobile phone. These variants can be advantageous for marketing, cost and practicality reasons.

The set of detection devices 11 can comprise broadband gas detection devices 11a.

The broadband gas detection devices 11a can be configured to analyze the air exhaled by the subject 300 and provide a signal associated with the concentration of one or more volatile substances present in the exhaled air.

The broadband gas detection devices 11a can be non-specific for a single molecule or for a single pathology.

The gas detection devices 11a can be sensors and/or transducers for gasses and/or volatile organic substances, such as sensors for CO, CO2, O2, NO, NO2, O3, SO2, hydrogen sulfide, irritating gases, reducing gases, oxidizing gases, total VOCs, methanol, acetone, phenols, or suchlike, of the electrochemical, catalytic, semiconductor, optical type or suchlike. For example, the sensors can be MOS (Metal Oxide Semiconductor) sensors, which advantageously are robust, even in the presence of elements such as siloxanes, and have an accuracy sufficient to measure concentrations of volatile substances in the order of ppb (parts per billion).

In one preferred embodiment, the gas detection devices 11a are selected from a group comprising sensors for measuring total VOCs and equivalent CO2 (value obtained from the measurement of molecular hydrogen) such as SGP30 by Sensirion, SCD30 by Sensirion for CO2, triple sensor for CO, Ammonia-H2-Ethanol-Methane-IsoButane-Propane, NO2 such as MiCS-6814 by SGX, ZE08-CH2O by Winsen for formaldehyde, sensors for measuring particulates PM0.5 PM1 PM2.5 PM4 PM10 such as SPS30 by Sensirion, 3SP_CO_1000 Package 110-102 by SPEC for CO, 3SP H2S_50 Package 110-304 by SPEC for H2S, 3SP_NO2_5F P Package 110-507 by SPEC for NO2, 3SP_{_}O3_20 C Package 110-407 by SPEC for O3, RESP_IRR_20 by SPEC for gas irritants NO2-O3-Cl2-SO2-H2S, 3SP_SO2_20 P Package 110-601 by SPEC for SO2, ZMOD4510 by Sensirion for NOx, O3, ZMOD4410 by Sensirion for TVOCs, equivalent CO2, sulfur-based odors, total odors, TGS81000 by Figaro for various contaminants in air, iAQ-Core by AMS for TVOCs, equivalent CO2, AGS01DB by ASAIR for VOCs, TGS2602-B00 by Figaro for VOCs and odors, CCS811B-JOPR5K by ScioScience for VOCs, TVOCs, equivalent CO2, BME680 by BOSH SENSORTEC for VOCs, Temperature, Humidity, Pressure, BME688 by BOSH SENSORTEC for VOCs, Temperature, Humidity, Pressure, SCD40 by Sensirion for CO2, XENSIV PAS CO2 by Infineon for CO2 and/or SGP40 by Sensirion for TVOCs.

According to some embodiments, the identification apparatus 10 can further comprise detection devices for monitoring breathing 11b.

The detection devices for monitoring breathing 11b can be selected from a group comprising acoustic sensors, flow and/or flow rate sensors, pressure sensors, sensors for detecting the motion of the rib cage of the subject 300, temperature sensors, humidity and/or fine particulate PM sensors.

In the text, we will refer to gas detection devices 11a and detection devices for monitoring breathing 11b, collectively referred to as detection devices 11.

The detection devices 11 can further comprise temperature sensors and/or humidity sensors, or use the temperature sensors and/or humidity sensors 11b, to correct the signals supplied by the gas detection devices 11a, which are also usually sensitive to these physical quantities.

In particular, the set of detection devices 11 comprises at least two of either detection devices 11a, 11b of a different type and/or detection devices 11a, 11b configured to function in different operating conditions.

The processing module 12 can be trained to analyze at least the signals as above and to synthesize an indicator 19a of the classification of the subject 300 in a class, among a set of classes to which the pathological state belongs.

The classes of belonging identify levels of severity relating to the pathological state with respect to which a diagnosis is to be provided for the subject 300. The classes of belonging can be at least two, for example "Healthy" and "Sick". As a further example, in the case of COVID19, the set of classes of belonging can be "Healthy", "Asymptomatic patient", "Symptomatic patient, "Cured".

According to some embodiments, the processing module 12 can comprise identification algorithms based on artificial intelligence, such as neural networks, SVM (Software Vector Machine), neuro-fuzzy networks, genetic algorithms or suchlike. Preferably, the data processing module 12 can comprise a neural network.

In a preferred embodiment, in order to exploit the evolution and the level of sophistication reached by neural networks for image recognition, the processing module 12 can comprise algorithms based on the state of the art of deep convolutional networks. For example, it can comprise algorithms based on deep convolutional networks studied for image classification, such as EfficientNet, MobileNet, ResNet or VGG16 or suchlike. This allows to identify, on each occasion, the most suitable network architecture according to accuracy and computational cost requirements. Taking advantage of the networks mentioned here also allows to take advantage of using pre-trained networks to apply transfer learning techniques, and therefore reduce the size of the dataset required for training.

According to some variants, in the identification apparatus 10 for training and data collection from breathing only, the processing module 12 may not comprise the identification algorithms based on artificial intelligence as above. The processing module 12 may also not comprise hardware suitable to execute the identification algorithms. Since the training can be carried out after having acquired the data, and off-line, as on a distributed electronic architecture 60, for example implemented with elements in the Cloud 68, in this identification apparatus 10 for training and data collection only, a processing module 12 can be used comprising processors of limited power, of varying technology and class, for example ARM or x86, and class of the following types: Cortex A8 TI AM335x or Intel Atom E3827 or Atom E3845.

The processing module 12 can comprise a programmable processing unit and a data storage module, not shown in the drawings.

The programmable processing unit can be any form of computer processor whatsoever usable in the field of computer technology, advantageously in the field of processing signals acquired by detection devices 11.

The programmable processing unit can comprise a prediction module, for using the identification algorithms based on artificial intelligence.

The storage module can be connected to the programmable processing unit and be among those commercially available, such as a random-access memory (RAM), a read-only memory (ROM), a floppy disk, a hard disk, a non-volatile memory (NOR FLASH and NAND FLASH), mass memory or any other form whatsoever of digital storage or electronic database.

The storage module can be able to store one or more identification algorithms executable by the programmable processing unit to obtain an indicator 19a of the classification of the subject 300 in a class among a set of classes of belonging of the pathological state.

The storage module can be able to store, at least temporarily, the signals as above acquired by the detection devices 11, or pre-processed versions thereof.

According to some embodiments, the communication module 13 is configured to send at least the signals as above and possibly the classification indicator 19a to an external distributed electronic architecture 60. It can also be configured to update the processing module 12.

The updating of the processing module 12 can comprise loading new identification algorithms, or re-trained identification algorithms, such as for example a new neural network trained offline by means of the new data collected in the field.

The identification apparatus 10, in use in the field, in addition to the operations described here, can be configured to continue to collect and send data to the distributed electronic architecture 60, for example in the Cloud 68.

According to some embodiments, the data can be labeled, making the information of the subject 300 who undergoes the test anonymous.

For example, if an alarm threshold is exceeded and an exam with higher accuracy were required, it can be provided to update the data on the Cloud 68 with the ground truth derived from these exams, and then include this data in the training set of the identification algorithm so as to be able to improve its diagnosis capacity and/or compensate for the domain shift (the "signature" of the virus on the recorded data). It should be noted that this continuous update procedure based on the Cloud 68 allows to follow possible mutations of the virus that could modify the domain shift.

The communication module 13 can comprise an interface module 14, a temporary archive 15, a publication server 16, and an interface 17 toward the distributed electronic architecture 60.

The interface module 14 can be a MODBUS module, a Python module or suchlike. For example, the MODBUS protocol, applied on a serial or Ethernet line, simplifies the interfacing with hardware already equipped with this protocol, since it is a standardized protocol. The communication module 13 can conveniently be configured so as to enable a traffic of at least 1 kbyte/sec, which is widely available in most of the communication modules currently on the market. The data saving module 69 can be present as an alternative or to support the communication module 13, so as to store the acquired data. The data storage module 69 can comprise a random-access memory (RAM), a read-only memory (ROM), a floppy disk, a hard disk (HARD DISK), a non-volatile memory (NOR FLASH and NAND FLASH), a mass memory or any other form of digital storage or electronic database.

The data saving module 69 can also comprise a physical interface for data retrieval. The physical interface can be a USB interface, a device for reading/writing optical discs, or other.

In the absence of connectivity, for example, the transfer of signals and information to and from the training distributed electronic architecture 60 can be carried out manually by a user or operator, by using a removable data storage device (for example a memory on a USB interface) as a temporary data transfer memory.

The identification apparatus 10 can comprise a containing chamber 20 for the air exhaled by the subject 300, in which at least the set of gas detection devices 11a is positioned.

According to some embodiments, the detection devices 11 can be disposed to occupy the entire area of the containing chamber 20 and be simultaneously subjected to the flow of exhaled air.

Alternatively, the detection devices 11 can be disposed sequentially in the transverse ducts of the chamber 20, so as to be subjected to the exhaled air according to a predefined sequence.

The containing chamber 20 can comprise an inlet and an outlet for the air. According to some embodiments, the containing chamber 20 can comprise air passage ducts. In particular, the inlet duct can be in communication with an air inlet hole 66.

According to some embodiments, the identification apparatus 10 can comprise an air insufflation element 67, for example a duct with a mouthpiece or a duct with a mask, to allow the subject 300 to exhale the air directly inside the containing chamber 20. In one variant, the air can be blown into an external container, for example a plastic container, a cylinder or other similar element, and then be sent to the detection devices 11 through the inlet hole 66.

In a known manner, the identification apparatus 10 can comprise devices for moving the air.

The identification apparatus 10 can comprise a system for cleaning the chamber 20 of the air exhaled by the subject 300, in order to prevent contamination between one subject 300 and the next, and errors in identifying the pathological state. For example, there can be provided clean air or gas cylinders for washing, devices for moving the air, disposable antiviral and/or antibacterial filters and suchlike.

In a known manner, the identification apparatus 10 can present a module for pre-treating 21 and conditioning the exhaled air. The module for pre-treating 21 the exhaled air can comprise devices for modifying its temperature, for example heating devices, the humidity rate, for example a dehumidification or humidification device, the pressure and/or suchlike, not shown in the drawings. Furthermore, the pre-treatment module 21 can be differentiated according to whether the pre-processed flow of exhaled air is sent to gas detection devices 11a or breathing monitoring devices 11b.

For example, suitable temperature values can highlight particular characteristics of the exhaled air, or fingerprint.

Advantageously, according to some embodiments, the pre-treatment/conditioning of the exhaled air can be carried out globally on the entire flow of exhaled air at entry, or locally for a single detection device 11 or set of detection devices 11.

The exhalation event of the subject 300 usually has a duration limited to a few seconds. Furthermore, the repetition of exhalation events in sequence leads to portions of exhaled air with different information contents. Finally, for respiratory diseases the subject 300 may not be able to repeat exhalation sequences. For these reasons, it is very useful to provide mechanisms that allow to analyze exhaled air in different physical conditions but contextually to a single exhalation event. Possible advantageous embodiments of the pre-treatment module 21, in combination with the detection devices 11, can make this functionality possible.

According to some example embodiments, the module 21 for pre-treating/conditioning the exhaled air can be configured to separate a single flow of the exhaled air into separate flow portions.

In this case, the pre-treatment module 21 can comprise a duct which has an inlet and two or more outlets.

Alternatively, the module can have containing elements, for example chambers, bags or suchlike. These containing elements can be able to contain flows of air acquired at different times. For example, in the same exhalation act, the initial part of the flow of air can be sent to one chamber and the end part into a different chamber. The separation of the air can take place by activating, for example sequentially, appropriate valves, for example non-return valves.

Each flow portion can be heated to a different temperature to that of the other portions.

Advantageously, in some embodiments, the identification apparatus 10 can have two or more copies of a same set of detection devices 11, obtaining what can be called an "array of sensor arrays" of the same type. For example, the identification apparatus 10 can have n identical modules, or sets, consisting of m detection devices 11, for example for VOCs.

Each flow can act on one set of detection devices 11.

According to some embodiments, the gas detection devices 11a, of the broadband type and not temperature compensated, can generate different data on each set, contributing with a greater amount of data to provide a characterization of the flow of exhaled air. Advantageously, the training of the identification algorithms can therefore make use of more information and converge more quickly to an effective diagnosis. Using copies of a same set of detection devices 11 can also reduce overall costs.

According to some embodiments, the pre-treatment module 21 can be programmable, for example on the basis of the clinical findings and/or the results of the re-training of the identification algorithms.

The pre-treatment module 21 can also be updated remotely.

The apparatus can comprise at least one device 18 for pre-processing the signals provided by the detection devices 11.

The pre-processing device 18 can be configured to select and/or filter the signals, or the signal parts, with useful information content, in order to reduce the set of training data and in general to improve the convergence of the networks' learning with better probability figures for recognition and diagnosis.

The pre-processing device 18 can be configured to normalize the input data coming from the detection devices 11. Advantageously, since the input quantities do not have the same range of values or the same units of measurement, a normalization process can prevent the identification algorithm from learning giving less importance to the characteristics represented with ranges of lower values. A normalization carried out by the pre-processing device 18 can also prevent the time and resources required to train the identification algorithm from increasing, in the event that the identification algorithm has to apply compensations in order to carry out a normalization autonomously.

For example, parts of the signal corresponding to a desired interval of the exhalation can be selected, signals with different sampling frequencies can be sampled in order to reduce the amount of data and the necessary computational and storage cost, the signals can be interpolated, combined with each other, or similar operations.

The pre-processing device 18 can be configured to identify and extract relevant characteristics of the signal to be used as an additional input to the identification algorithm. This allows to simplify the implementation and/or to speed up the processing. For example, for physical signals relating to the exhaled air, it is possible to automatically identify temporal and characteristic markers of the profile of the exhaled air.

Advantageously, the identification of the pathological state, that is, the fingerprint of the pathological state, can in this way also be detected by means of the dynamics of the evolution of the signals provided by the detection devices 11.

In a preferential form, the pre-processing device 18 can be configured to pre-process the signals of the detection devices 11 so that they can be represented, as images, in two dimensions and the processing module 12 can be trained to recognize images of objects.

One mode for transforming into 2D images the time sequences that represent the evolution of the detection devices 11 during the patient's exhalation can be achieved, for example, by building a matrix in which each column contains the data of all the detection devices 11 at the time t, and the subsequent column contains the data of all the detection devices 11 at the time t+1, for example one column for every second, every half-second or suchlike. If a color is associated with each value, for example in shades of gray, with the appropriate pre-processing to expand the possible range of values of each detection device 11 within the range that can be represented in shades of gray, for example between 0 and 255, the matrix created is a grayscale image relating to a single exhalation of the patient. The identification algorithm can then be trained with the images relating to all subjects 300.

By way of example, the signals can be processed by means of domain change techniques applied to a single signal, using spectrograms and Fourier transforms, or to multiple signals, for example by showing the entire information content of the set of detection devices 11 in a single image.

The identification apparatus 10 can comprise a local interface 19 to display the classification indicator 19a and/or to instruct the subject 300 on how to exhale the air during one or more exhalation steps.

The local interface 19 can comprise a display, a monitor or suchlike.

The local interface 19 can further comprise a keyboard, or other input device, to allow an operator to enter information relating to the subject 300, such as identification data, information relating to the test performed, such as date and time of execution, medical history and/or suchlike.

According to some embodiments, the platform 50 for identifying pathological states comprises a distributed electronic architecture 60 for training and data management and one or more apparatuses 10 for identifying pathological states.

The electronic architecture 60 can comprise one or more data management elements 61, a device 62 for centralizing information and a module 63 for training algorithms which are based on artificial intelligence.

The management elements 61 can be configured to acquire data from the one or more identification apparatuses 10 and to store them. The management elements 61 can comprise a programmable processing unit, a data storage module, an interface for communication with the identification apparatus 10 as previously described, and interfaces for communication with the Cloud 68, not shown in the drawings. The management elements 61 can also be configured to display the signals and/or indicators provided by the identification device 10. The management elements 61 can also be configured for inputting further information, such as a possible diagnosis, possibly in cooperation with an acquisition device 65, or a medical history or suchlike.

For example, one management element 61 can be a tablet or a personal computer on the display or monitor of which the signals and/or indicators can be displayed and by means of which information can be inputted.

In one preferential form that allows the autonomous communication of the identification apparatus 10 with the distributed electronic architecture, the management element 61 is integrated in the apparatus 10 itself.

The information centralizing device 62 can be configured to acquire data and information, such as signals and/or indicators sent by the management elements 61, information sent by an external data archive 64 or by the acquisition device 65, in order to manage them and send them to the training module 63.

The information centralizing device 62 can comprise a programmable pre-processing unit 62a of the signals at input from 61, 64 and 65.

The programmable pre-processing unit 62a can be configured, for example, for a pre-processing of the data, which is usually useful in the training step, since the training can require high computing power. As a further example, it can be configured to experiment with variations to the training and/or identification procedure of the identification algorithm. The information centralizing device 62 can comprise a programmable processing unit and a data storage module, not shown in the drawings.

The training module 63 can comprise a programmable processing unit and a data storage module, not shown in the drawings. In order to reduce training times, the module 63 can comprise a parallel computing unit, such as for example a Graphics Processing Unit (GPU).

The training module 63 can comprise interfaces toward an operator, not shown in the drawings, for example an output device, such as a screen or video terminal and/or a keyboard or other input device or other user interface, including of the remote type, such as for example a web-based graphic interface, a character-oriented terminal or suchlike, to allow the operator to carry out the training method 100 of the algorithm based on artificial intelligence in a known manner.

For example, the learning can take place in a supervised mode: a representation is obtained for each exhalation session, for example as a 2D matrix in color or shades of gray, and it is associated with its label (asymptomatic patient, symptomatic patient, recovered, never sick, healthy or suchlike), known at the time of examination or later. These images or representations, associated with their diagnostic meaning, that is, with the label as above, are used to train the identification algorithm according to known methodologies.

For example, in order to reduce the amount of data necessary for the identification algorithm to reach a sufficient degree of accuracy, it is possible to apply transfer learning techniques. In the case in question, it is possible to re-train identification algorithms already trained for image recognition and exploit their previous knowledge, further specializing them on the data from the breathing of possible patients.

Furthermore, with a view to continuous improvement and to counter the effects of possible changes in the effects on breathing, caused for example by mutations of the virus in the case of Covid, or of bacteria, for example in Tuberculosis, or in other diseases, it can be possible to continue to train the identification algorithm with the data coming from the exhalations, exploiting the connection with the Cloud 68 to gather and label the new data collected. The continuous training produces new trained identification algorithms that can be once again loaded into the identification apparatuses in the field.

The training module 63 can also comprise a graphics interface, which in particular can have a Graphics Processing Unit (GPU) and a graphics software module for monitoring the training procedure.

The graphics training module can be configured to cooperate with the output or input devices to allow the operator to input, manage and view data relating to the training of the algorithm.

The electronic architecture 60 can comprise the external data archive 64. The data archive 64 can comprise data relating to other potentially useful information, such as diagnoses and medical histories relating to other subjects, diagnoses obtained with other types of instruments or carried out by medical personnel 400, statistical data and/or suchlike. According to one variant, the electronic architecture 60 can cooperate with a data archive outside the platform 50, and the data can be stored on third-party databases for which the platform is enabled for reading access.

The electronic architecture 60 can comprise a device 65 for acquiring information relating to the subjects 300 tested by means of the identification apparatus 10. The acquisition device 65 can be a computer, a server, a tablet or any other device whatsoever equipped with a processing unit and a storage unit for acquiring and saving the information as above. The information as above can comprise a diagnosis relating to the subjects 300 tested, carried out by medical personnel 400 independently or by means of further instrumentation, for example laboratory instrumentation. It can also comprise data relating to the subjects 300, a history of previous pathologies and/or suchlike.

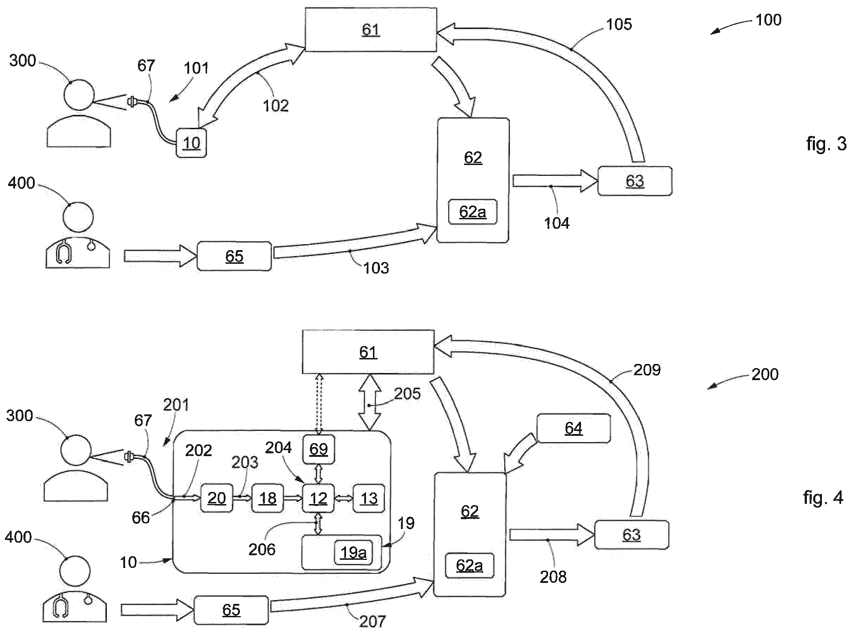

According to some embodiments and as shown in fig. 3, the method 100 for training an apparatus 10 for identifying pathological states comprises at least:
- identifying a group of subjects 300;
- receiving 101, in the identification apparatus 10, the air exhaled by each subject 300;
- putting the exhaled air in contact at least with the gas detection devices 11a;
- detecting signals produced at least by the gas detection devices 11a and associated with the concentration of one or more volatile substances present in the exhaled air;
- transmitting 102 at least the signals as above to a distributed electronic architecture 60 for training and data management;
- acquiring information 103 relating to each subject 300, associating it with the signals detected for the subject 300, which comprise at least one diagnosis relating to the classification of the subject 300 in a class among a set of classes to which the pathological state belongs;
- sending 104 the signals and information as above to a module 63 for training algorithms which are based on artificial intelligence and processing them for the training of an algorithm that identifies pathological states;
- updating 105 the processing module 12 by means of the trained identification algorithm.

The transmitting 102 to the electronic architecture 60 can comprise the step of transmitting the classification indicator 19a, useful for statistical analysis and for continuous re-training.

The acquiring information 103 relating to a diagnosis can be carried out, during the acquisition for the training, with subjects 300 whose diagnosis is already known with a medical procedure not assisted by the invention and medical history, or that is not yet known and will be disclosed later, with a diagnosis from medical procedure not assisted by the invention and medical history. Advantageously, this information can be used, in a known manner, by the training module 63 to train the identification algorithm.

The acquiring information 103 can provide to also associate additional medical history information with the diagnosis. Advantageously, such additional medical history information can be used for a better diagnosis and/or treatment.

The training method 100 can provide to define the number of subjects 300 to be tested for the training of the identification apparatus 10. For example, the number of subjects can be sufficient to form training sets, or training sets and validation training or suchlike.

The training method 100 can comprise acquiring additional information from an external data archive 64.

The training method 100 can comprise, for the training of the identification algorithm, to acquire and update a pre-existing algorithm based on artificial intelligence related to a different pathological state. For example, a neural network already trained to carry out a diagnosis of the breathing of possible tuberculous patients can be used as a starting network to re-train it for diagnosing COVID-19 patients. The state of the art justifies advantages in using this technology. Advantageously, in this way it is possible to use known algorithms based on artificial intelligence, already in use and economical, thus reducing development times and costs and increasing the reliability of the trained algorithm. For example, transfer-learning techniques can be used on algorithms already trained with other data sets.

The training method 100 can comprise pre-processing the signals of the detection devices 11, so as to be represented, as images, in two dimensions, and training the identification algorithm to recognize images.

According to some embodiments and as shown in fig. 4, the method 200 for identifying pathological states in a subject 300 by means of an apparatus 10 for identifying pathological states can comprise at least:
- training a data processing module 12 by means of an identification algorithm or verifying that the data processing module 12 has been trained;
- receiving 201, in an identification apparatus 10, the air exhaled by the subject 300;

- putting in contact 202 the exhaled air at least with broadband gas detection devices 11a;
- detecting 203 signals produced by the gas detection devices 11a and associated with the concentration of one or more volatile substances present in the exhaled air;
- processing 204 the signals, by means of the trained data processing module 12, in order to obtain an indicator 19a of the classification of the subject 300 in a class among a set of classes to which the pathological state belongs;
- transmitting 205 to a distributed electronic architecture 60 at least the signals and the classification parameter.

The identification method 200 can comprise making available 206 the classification indicator 19a by means of a local interface 19 for displaying the classification indicator 19a.

The identification method 200 can comprise sending 208 the signals and information as above to a module 63 for training algorithms which are based on artificial intelligence and processing them for the training/re-training of an algorithm for identifying pathological states.

The identification method 200 can comprise updating 209 the data processing module 12 by means of the re-trained identification algorithm.

The identification method 200 can comprise acquiring information 207 relating to each subject 300. The acquiring information 207 can provide to acquire, during the identification of the pathological state, with subjects whose diagnosis is not known, additional medical history information. Advantageously, such additional medical history information can be used for better diagnosis and/or treatment.

The identification method 200 can comprise using a same identification apparatus 10 both for the training and also for the identification of pathological states.

According to some variants, the identification method 200 can comprise the step of using different apparatuses for the training and for the identification of pathological states. For example, a first identification apparatus 10 can be used to collect data from the breathing, while a second identification apparatus 10 can be used for identification and diagnosis only. Advantageously, the identification method 200 can provide to use a second identification apparatus 10 having a simplified acquisition and communication part.

In addition, or alternatively, the identification method 200 can provide to use a second identification apparatus 10 which has a simplified contagion protection part, which can be used as a personal device for use similar to a mobile telephone.

The training method 100 and the identification method 200 can also comprise analyzing the physical characteristics of the exhaled air, selected from a group comprising a duration, an exhalation flow or exhalation profile of the subject 300.

The training method 100 and the identification method 200 can comprise instructing the subject 300 on how to exhale the air during one or more exhalation steps. Including, in the acquisition protocol (both for the training step and also for the diagnosis step), a guidance system on how to exhale can be decisive for the optimal acquisition of data on the exhaled air.

The training method 100 and the identification method 200 can comprise automatically updating the system for guiding the subject 300 with the exhalation (procedure, intensity, duration) on the basis of one or more analyzes, in progress or previously carried out by the identification apparatus 10 and stored, and/or indications from medical personnel 400.

The training method 100 and the identification method 200 can comprise pre-treating/conditioning the exhaled air.

The training method 100 and the identification method 200 can comprise a customization by classes of subjects 300.

It is clear that modifications and/or additions of parts or steps may be made to the apparatus 10 for identifying pathological states, to the platform 50 for identifying pathological states, to the method 100 for training the identification apparatus 10 and to the method 200 for identifying pathological states as described heretofore, without departing from the field and scope of the present invention as defined by the claims.

## Claims

1. Apparatus for identifying pathological states of a subject by means of breath analysis, comprising at least:
- a set of detection devices selected from broadband gas detection devices configured to analyze the air exhaled by the subject and provide signals associated with the concentration of volatile substances present in the exhaled air and/or detection devices for monitoring breathing;
- a chamber for containing the air exhaled by the subject, in which at least the set of gas detection devices is positioned, said containing chamber comprising an inlet and an outlet for said air;
- a module for processing at least said signals which can be trained by means of artificial intelligence techniques to synthesize an indicator of the classification of the subject in a class, among a set of classes to which the pathological state belongs; and
- a module for communicating data to a distributed electronic architecture for sending at least said signals and possibly said classification indicator and for updating the processing module and/or a data saving module, alternatively or as a support to the data communication module, able to store the data;
**characterized in that** said set of detection devices comprises at least two of either detection devices of a different type and/or detection devices configured to function in different operating conditions.

2. Apparatus as in claim 1, **characterized in that** said processing module is trained to recognize the fingerprint of the pathological state.

3. Apparatus as in claim 1, **characterized in that** the apparatus comprises two or more copies of a same set of detection devices.

4. Apparatus as in claim 1, **characterized in that** the apparatus comprises a module for pre-treating the air exhaled by the subject **and in that** the module for pre-treating the air exhaled by the subject comprise devices selected from devices for modifying the temperature of the air, devices for modifying the humidity rate of the air and devices for separating the flow of exhaled air into separate flows.

5. Apparatus as in claim 1, **characterized in that** the gas detection devices are selected from a group comprising volatile organic matter sensors and gas sensors.

6. Apparatus as in claim 1, **characterized in that** said detection devices for monitoring breathing selected from a group comprising acoustic sensors, flow and/or flow rate sensors, sensors for detecting the motion of the rib cage of the subject, temperature sensors and/or humidity sensors.

7. Apparatus as in claim 1, **characterized in that** the apparatus comprises at least one device for pre-processing the signals provided by said detection devices.

8. Apparatus as in claim 1, **characterized in that** said pre-processing device is configured to automatically identify and extract temporal and characteristic markers of the exhaled air.

9. Apparatus as in claim 7 or 8, **characterized in that** said pre-processing device is configured to pre-process the signals of said detection devices so that they can be represented, as images, in two dimensions and said data processing module can be trained to recognize images of objects.

10. Apparatus as in claim 1, **characterized in that** the data processing module which can be trained by means of artificial intelligence techniques comprises a neural network, **in that** said neural network can be trained with images **and in that** said neural network is a deep convolutional network selected from EfficientNet, MobileNet, Imagenet, ResNet or VGG16.

11. Apparatus as in claim 1, **characterized in that** the apparatus comprises a local interface for displaying the classification indicator and/or instructing the subject on how to exhale the air during one or more exhalation steps.

12. Platform for identifying pathological states, **characterized in that** it comprises a distributed electronic architecture for training and data management and one or more apparatuses for identifying pathological states comprising at least a set of broadband gas detection devices, a data processing module which can be trained by means of artificial intelligence techniques and a module for communicating data to said electronic architecture and/or a data saving module, the electronic architecture comprising at least one module for training algorithms which are based on artificial intelligence.

13. Identification platform as in claim 12, **characterized in that** the electronic architecture comprises a data archive and/or cooperates with an external data archive and comprises a device for acquiring information relating to the subjects tested by means of said one or more apparatuses for identifying pathological states.

14. Method for training an apparatus for identifying pathological states **characterized in that** it comprises at least a set of broadband gas detection devices, a data processing module which can be trained by means of artificial intelligence techniques and a module for communicating data to a distributed electronic architecture for training and data management and/or a data saving module, the method comprising at least:
- identifying a group of subjects;
- receiving, in the apparatus for identifying pathological states, the air exhaled by each subject;
- putting the exhaled air in contact at least with the gas detection devices;
- detecting signals produced at least by the gas detection devices and associated with the concentration of one or more volatile substances present in the exhaled air;
- transmitting at least said signals to a distributed electronic architecture for training and data management and/or saving the data for transmission;
- acquiring information relating to each subject, associating it with the signals detected for the subject, said information comprising at least one diagnosis relating to the classification of the subject in a class among a set of classes to which the pathological state belongs;
- processing said signals and information by means of a module for training algorithms which are based on artificial intelligence for the training of an algorithm that identifies pathological states;
- updating the data processing module by means of the trained identification algorithm.

15. Training method as in claim 14, **characterized in that** it comprises acquiring further information from an external data archive.

16. Training method as in claim 14, **characterized in that** it comprises, for the training of said algorithm that identifies pathological states, acquiring and updating a pre-existing algorithm based on artificial intelligence relating to a different pathological state.

17. Training method as in claim 14, **characterized in that** it comprises pre-processing the signals of the detection devices so that they can be represented, as images, in two dimensions and training the identification algorithm to recognize images.

18. Training method as in claim 14, **characterized in that** it also comprises analyzing the physical characteristics of the exhaled air, selected from a group comprising a duration, an exhalation flow or exhalation profile of the patient, **and in that** it comprises instructing a subject on how to exhale air during one or more exhalation steps.
